# EUROPEAN PATENT APPLICATION

(11) **EP 3 906 965 A1**
(43) Date of publication of application: **10.11.2021**
(21) Application number: 21170024.0
(22) Date of filing: 22.04.2021
(51) Int. Cl.: A61N 1/36, A61N 1/05

(54) **A NEUROMODULATION SYSTEM FOR PLANNING AND/OR ADJUSTING AND/OR PROVIDING A NEUROMODULATION THERAPY**

(30) Priority: 23.04.2020 EP 20020190
(71) Applicant: ONWARD Medical B.V., 5656 AE Eindhoven (NL)
(72) Inventor: Caban, Miroslav, 5656AE Eindhoven (NL); Zitzewitz, Joachim, 5656AE Eindhoven (NL); Delattre, Vincent, 5656AE Eindhoven (NL); Courtine, Grégoire, 1003 Lausanne (CH); Komi, Salif, 1012 Lausanne (CH); Demesmaeker, Robin, 1110 Morges (CH); Wagner, Fabien, 33 000 Bordeaux (FR)
(74) Representative: DTS Patent- und Rechtsanwälte Schnekenbühl und Partner mbB

(57) **Abstract**

A neuromodulation system (10) for planning and/or adjusting and/or providing a neuromodulation therapy, comprising:
- at least one neuromodulation means (12) configured to provide neuromodulation at least partially by means of neurostimulation;
- at least one neuromodulation controller (14) configured to control the neuromodulation means (12),
wherein the neuromodulation controller (14) is further configured to control the neuromodulation means (12) at the beginning of a neuromodulation action including neurostimulation that the neurostimulation comprises a starting sequence and/or at the end of a neuromodulation action including neurostimulation that the neurostimulation comprises an ending sequence, wherein the neuromodulation action is planned to be provided and/or provided to a neuronal population (NP) of a patient for neuronal population polarization, and wherein the neuronal population polarization during the starting sequence and/or ending sequence is limited compared to the neuronal population polarization during the stimulation after the starting sequence and/or before the ending sequence.

## Description

The present invention relates to a neuromodulation system for planning and/or adjusting and/or providing a neuromodulation therapy for a patient.

The present invention further relates to the use of a neuromodulation system for planning and/or adjusting and/or providing a neuromodulation therapy for a patient in a method for the treatment of a patient.

The spinal cord is an integral part of the central nervous system (CNS). Spinal cord injury (SCI), but also other disorders (e.g. stroke, multiple sclerosis, autonomic failure, autonomic neuropathy or cancer of the neurological tissue which impair operation of descending sympathetic pathways that normally facilitate control of autonomic functions) result in motor deficits. For instance, SCI interrupts the communication between the spinal cord and supraspinal centres, depriving these sensorimotor circuits from the excitatory and modulatory drives necessary to produce movement. However, SCI results also in sensory deficits and in autonomic dysfunctions. In particular, SCI results in disconnection of some, most, or all descending sympathetic pathways that carry signals responsible for regulating arterial blood pressure, heart rate and/or gut and/or bladder function.

A neuronal population (NP) is a group of neurons. A neuronal population may include groups of neurons having homogeneous or weakly heterogeneous properties.

Spinal cord stimulation (SCS) by means of neuromodulation system/neurostimulation system is a well-established neuromodulatory/neurostimulatory therapy not only for restoring locomotion/motoric function after spinal cord injury or central nervous system diseases, but also for treating inter alia pain and/or restoring autonomic function.

A neuromodulation system, especially a neurostimulation system for a patient suffering from motoric dysfunction and/or autonomic dysfunction requires programming to define which stimulation settings need to be used to evoke certain muscles or muscle groups (including also smooth muscles). Such muscles and/or muscle groups may be responsible for locomotion of the arms or legs, and/or responsible for e.g. bowel movement, sphincter control, bladder control, respiratory function, blood pressure and/or sexual function.

Neuromodulation, in particular neurostimulation, is typically applied to a subject by a neuromodulation system comprising at least one electrode array comprising at least one electrode and a pulse generator.

The electrode array, e.g. comprised in a lead paddle, can be applied for percutaneous electrical stimulation, transcutaneous electrical nerve stimulation (TENS), epidural electrical stimulation (EES), subdural electrical stimulation (SES), functional electrical stimulation (FES) and/or all neurostimulation and/or muscle stimulation applications that use at least one electrode array and/or at least one electrode.

EES shows promising results for spinal cord injury therapy to restore motor function. The mechanisms are still unclear and under investigation, but EES can both stimulate the leg muscles through the proprioceptive afferent fibers and restore the neuronal network in the spinal cord. EES uses a multi-electrode array placed on the dorsal side of the spinal cord on top of the dura matter. In rats, the combination of serotonergic agonists and EES was able to acutely transform spinal networks from non-functional to highly functional and adaptive states as early as 1 week after injury ***(***Courtine G, et al., Transformation of nonfunctional spinal circuits into functional states after the loss of brain input. Nature neuroscience 12, 1333-1342, (2009*)).* Moreover, EES also restores voluntary control of locomotion by rewiring the injured spinal cord area ***(***Wenger N, et al., Spatiotemporal neuromodulation therapies engaging muscle synergies improve motor control after spinal cord injury, Nature Medicine 22, 138-145 (2016))*.* Because of the complexity of the spinal cord, delivering EES stimulation on the multi-electrode array (lead) implanted is quite challenging.

Once stimulation parameters have been found to stimulate certain muscles (or groups of muscles), the muscular activations need to be sequenced in time. For example, walking is defined by alternating stimulations of specific muscles on the right and on the left leg. Similarly, any kind of other locomotion, e.g. running, swimming, cycling, rowing, can be thought of as a timeline or sequence of muscular stimulations.

During a neuromodulation session, stimulation may be turned on and off in order to support the movement of certain targeted muscles. A stimulation block may comprise a pulse train for, e.g. a certain target muscle and/or group of muscles. When stimulation is turned on, no matter the frequency of stimulation, the first pulse often represents a shock to the system (and/or the patient). The first pulse of the stimulation block is a sudden input to a system otherwise in a balanced regime. Once the stimulation continues, the system (and/or the patient) adapts to this new input and the resulting functional support of stimulation is observed.

The response (first reaction to the shock stimulation) of the patient may be non-physiological and may cause a non-natural contraction, which may lead to erratic movements and/or strong initial accelerations at the onset of stimulation.

The non-physiological response to a stimulation block and/or the (first) pulses of a stimulation block may be explained by the fact that during the transition between a non-stimulating and a stimulating state, the neural circuitry involved is shocked by a sudden input. The input is sudden because of the way electrical stimulation activates neural fibers. For example, an amplitude may be increased from 0 mA and it is possible that no response is observed. At a given threshold, the neurons will be recruited and begin to fire. Naturally, the body uses a specific encoding in the peripheral neural signals. Typically, this means continuous changes in firing frequency, rather than discrete jumps. EES recruits the fibers involved in monosynaptic reflexes, which feed peripheral information into the spinal cord. However, since with EES these firing frequencies can be increased in a discrete manner, the system is subject to a non-natural change. The sudden increase is non-natural and thus the response is non-physiological.

Erratic movements may be observed during any motion and/or motion training during neuromodulation/ neurostimulation, e.g. during gait training, in particular e.g. during swing initiation and/or food touch down.

In particular, erratic movements may mean that the execution of the motion is not controlled by a patient treated with a neuromodulation system, but rather by the initial kick of the stimulation and biomechanics. For example, during swing initiation during gait training, this means that the knees of the patient may rise high, at an impressive (non-natural) speed. During foot touch-down, what can be noticed, is a weak control of where the foot hits the ground. Instead, the downwards motion is determined by biomechanics and gravity.
The erratic movements may also be caused by the way EES interacts with the CNS, however now on a larger scale. This stimulation may activate a neural pathway that shares circuits with a withdrawal reflex. The resulting muscle activations are somewhere between a withdrawal reflex and a known flexion muscle synergy. The observed muscle activations and/or motion are not exactly of one or the other, however, the reflex is indeed fast acting like withdrawal.

Further, erratic reactions of a subject stimulated may also be possible during stimulation to restore physiological/normal bowel movement, sphincter control, bladder control, respiratory function, blood pressure and/or sexual function.

Due to the effects described above, responses to neurostimulation, e.g. motion, is difficult to control for a patient treated with a neuromodulation/neurostimulation system. The recruitment of the flexion synergy is another example of the all-or-nothing effect of stimulation on recruitment of neural circuitry, however there is an interval between the motor threshold and functional threshold where the effect of stimulation can be controlled. Since the neural circuitry involves that of the withdrawal reflex, control on the speed of retraction can prove to be difficult.

It is an object of the present invention to provide a planning and/or control system for a neuromodulation system, especially a neurostimulation system for a patient that enables smooth movements of a patient, that could be comparable to a healthy subject.

This object is solved according to the present invention by a planning and/or control system for a neuromodulation system with the features of claim 1. Accordingly, a neuromodulation system for planning and/or adjusting and/or providing a neuromodulation therapy, comprising:
- at least one neuromodulation means configured to provide neuromodulation at least partially by means of neurostimulation;
- at least one neuromodulation controller configured to control the neuromodulation means,
wherein the neuromodulation controller is further configured to control the neuromodulation means at the beginning of a neuromodulation action including neurostimulation that the neurostimulation comprises a starting sequence and/or at the end of a neuromodulation action including neurostimulation that the neurostimulation comprises an ending sequence,
wherein the neuromodulation action is planned to be provided and/or provided to a neuronal population (NP) of a patient for neuronal population polarization,
further wherein the neuronal population polarization during the starting sequence and/or ending sequence is limited compared to the neuronal population polarization during the stimulation after the starting sequence and/or before the ending sequence.

Neuronal population polarization means an increase in cell potential.

The invention is based on the basic idea that with providing a starting sequence that is executed before a stimulation block the nerves and/or nervous system of the patient may not be shocked by sudden stimulation. Additionally, and/or alternatively, with providing an ending sequence that is executed after a stimulation block the stimulation of nerves and/or the nervous system of the patient may be tapered, such that a physiological response to the stimulation may be enabled, comparable to a healthy subject. In other words, the system enables stepwise and/or slowly approaching and/or ending the stimulation block, in order to enable controlled responses to the stimulation. With pulses that are of the same electrode configuration as the pulses of the stimulation block but provided before and/or after the patient is treated with the actual stimulation, the nerves and/or nervous system of the patient get adjusted to the following and/or ending stimulation. Overall, this may provide the effect that the neural circuitry of the patient is already adapted to stimulation by the onset of a stimulation block, and that unnatural responses, e.g. hectic movements, are avoided.

In general, the neurostimulation may comprise stimulation parameters, wherein the stimulation parameters comprise power, amplitude, current, voltage, pulse width, frequency and/or duration.

In particular, at least one stimulation parameter of the starting sequence and/or ending sequence may have a lower level compared to the at least one stimulation parameter of the normal stimulation (stimulation block/pulse train). This means that the starting sequence and/or ending sequence may be characterized by a lower power, amplitude, current, voltage, pulse width, frequency and/or duration as the normal stimulation. In particular, at least one pulse and/or pulse sequence of the starting sequence and/or ending sequence may be characterized by a lower power, amplitude, current, voltage, pulse width, frequency and/or duration as the pulses and/or pulse sequence of the normal stimulation.

It is possible that at least one stimulation parameter may be increased during the starting sequence and/or decreased during the ending sequence. In other words, the power, amplitude, current, voltage, pulse width, frequency and/or duration of at least one pulse and/or pulse sequence of the starting sequence and/or ending sequence may be increased and/or decreased.

In particular, the increase of the at least one stimulation parameter may be a linear, non-linear, exponential, polynomic and/or stepwise increase and the decrease of the at least one stimulation parameter is a linear, non-linear, exponential, polynomic and/or stepwise decrease. In other words, the power, amplitude, current, voltage, pulse width, frequency and/or duration of at least one pulse and/or pulse sequence of the starting sequence and/or ending sequence may be increased and/or decreased in a linear, non-linear, exponential, polynomic and/or stepwise fashion. Depending on the task to be performed/desired response to the stimulation, the type of increase and/or decease may be selected in order to enable physiological responses to the stimulation without erratic reactions.

Further, the starting sequence may include a pre-pulse (PP), wherein at least one stimulation parameter of the pre-pulse is of a lower level compared to the at least one stimulation parameter of a normal stimulation pulse.

In particular, a less powerful pulse may be understood as a pulse of less amplitude and/or lower pulse width.

In general, a pre-pulse may be of any amplitude lower or equal to the amplitude of the pulses of the stimulation block. For instance, in case of a single pre-pulse, the pre-pulse may be of 30 to 90%, preferably 50 to 80% of the functional stimulation amplitude, i.e. 30 to 90%, preferably 50 to 80% of the amplitude of the stimulation block that follows.

Alternatively, the at least one pre-pulse may be of the same power, amplitude, current, voltage, pulse width, frequency and/or duration compared to the stimulation pulse(s) of the stimulation block.

In general, a starting sequence may comprise at least one pre-pulse that is provided before a stimulation block comprising a pulse train is provided. In other words, a pre-pulse may be provided at any time before the beginning of the stimulation block. Preferably, the pre-pulse may be provided seconds, preferably milliseconds before the stimulation block is initiated.

It may be possible that the delay between the pre-pulse and the subsequent stimulation block can be different from the interpulse interval within the stimulation block (shorter or longer).

What is exploited physiologically with a pre-pulse (or pre-stimulus) is the fact that stimulation recruits monosynaptic reflexes and that these may be conditioned. Thus, reflexes may be prone to suppression if a previous pulse is applied. A single pre-pulse may thus suppress/condition the monosynaptic reflex for a given duration, meaning a stimulation during this subsequent time will have an effect different than expected. This conditioning may be achieved with a stimulus below threshold, thus not evoking a muscle twitch but still conditioning the circuit. When the functional stimulation block and/or pulse train then arrives in the circuit, the effect of sudden stimulation onset may be eliminated.

The starting sequence may comprise more than one pre-pulse. If this is the case, the pre-pulses may be in particular of lower frequency than the normal stimulation pulses of the pulse train of the stimulation block. In case of two or more pre-pulses, the second (and/or second plus x) pre-pulse may either be of the same power, amplitude, current, voltage, pulse width, and/or duration as the previous pre-pulse, or between the power, amplitude, current, voltage, pulse width and/or duration of the previous pre-pulse and the functional power, amplitude, current, voltage, pulse width and/or duration of the pulses of the stimulation block.

In terms of stimulation partiture tuning, the addition of the pre-pulse does not add any complexity. The pre-pulse may invariant with respect to different activities.

A starting sequence and/or ending sequence with more than one pre-pulse may also be understood as ramping. In other words, the starting sequence and/or the ending sequence may include a pulse ramping.

In particular, the pulse ramping may include a ramping up from a starting level of at least one stimulation parameter to a higher level of the at least one stimulation parameter used for the neurostimulation and/or a ramping down from a level of at least one stimulation parameter used for the neurostimulation to a lower level of the at least one stimulation parameter, e.g. below the threshold used for neurostimulation. In other words, the starting sequence and/or the ending sequence may include a pulse ramping. Pulse ramping may further allow to prevent the patient treated with the system from an initial shock of neurostimulation and/or aprupt end of neurostimulation.

The starting sequence may include a pulse ramping, wherein the pulse ramping may include a ramping up from a starting pulse energy level to a higher pulse energy level being used for the neurostimulation. Also, this may have the advantage that unnatural movements/responses to neurostimulation are avoided and/or reduced.

The starting sequence including pulse ramping may include at least one pulse.

The ending sequence including pulse ramping may include at least one pulse.

Ramping (up ramping and/or down ramping) of stimulation may be understood as before or during the execution of a stimulation block, the amplitude of pulses is progressively increased and/or progressively decreased. One effect is that the stimulation is progressively felt by the patient and so he can control it with better precision. Furthermore, the stimulation is better adapted to provide support at the beginning and at the end of a movement, where typically a lower amplitude is required, to enable smooth responses to stimulation, in particular smooth movements.

A progressive increase or decrease of the amplitude may make the stimulation act in a more natural manner. For instance, when a patient is stimulated during gait training, during an increase of amplitude (ramp up), the initial acceleration of the motion may be reduced, allowing the patient to better control the initial swing phase velocity. During a decrease of amplitude (ramp down), the stimulation support may not be abruptly stopped. This means that the patient stimulated by the system may have better control during gait training, in particular during swing phase/foot touch down.

Alternatively, when a patient is stimulated for restoring and/or controlling bowel movement, sphincter control, bladder control, respiratory function, blood pressure and/or sexual function, better control of sphincters and/or blood pressure and/or respiration and/or sexual function may be enabled.

The increase or decrease of the amplitude during up ramping or down ramping, respectively, may either be linear or non-linear linear (e.g. exponential, polynomic and/or stepwise). Amplitude increase or decrease may be defined either by a fixed number of pulses and/or a fixed number of time and/or a duration relative to the duration of a stimulation block.

Ramping pulses with a too low amplitude may not have any stimulation effect and therefore delay the stimulation by ***n*/*f*** seconds (n being the number of pulses without any effect, f being the stimulation frequency). E.g. in case of a stimulation frequency and ramping over five pulses of which the first three don't have any effect, a delay of 150 ms is obtained. This may also be the case without ramping as the first pulse may cause massive overshoot which may suppress the effect of the second pulse. The delay may to a certain extent be compensated by elongating the time of the stimulation block.

In general, the number of pulses of the ramping may be selected to minimize time delays to the stimulation block onset.

In particular, the ramping may differ from neuromodulation session to neuromodulation session. Thus, the ramping may be variable. The ramping may, for instance, be adapted to the rehabilitation progress of the patient treated with the system.

If two pulses are too close to each other (in time), the first pulse may suppress the second pulse.

The ramping may be defined for each task to be performed during neuromodulation/neurostimulation. For instance, the ramping may be defined for swing and stance phase during gait training, for walking (gait), running, swimming, cycling, rowing, stepping, standing up, sitting down, grasping, etc. Complex ramping may be used for tasks such as cycling e.g. a sinusoidal variation in amplitude, with flexion extension in anti-phase. The effect of the ramp up may be quantified by a measure of the acceleration following stimulation block onset, which may be lower with better ramping parameters.

The effect of the ramp down may be quantified by a measure of the control the patient exhibits during performing a task, such as foot touch down. For instance, this may be recorded either by measuring the deceleration of the foot (which should be less abrupt) or by the consistency of foot placement.

Alternatively, the ramping may be defined for controlling bowel movement, sphincter control, bladder control, autonomic function, respiratory function, blood pressure and/or sexual function.

The ramping sequence may comprise any number of pulses.

The system may further comprise at least one of a processor, a telemetry module, a feedback module, a sensor, a sensor network.

The level of at least one stimulation parameter of the starting sequence and/or ending sequence may be determined manually and/or automatically based on response data.

Further, the starting sequence and/or ending sequence may comprise a pre-warning signal and/or stop-warning signal. Alternatively, the starting sequence and/or ending sequence may be accompanied by a pre-warning signal and/or stop-warning signal.

The pre-warning signal and/or stop-warning signal may be an acoustic, visual, haptic, electrical, sensory and/or temperature signal. The pre-warning signal may help the patient to adjust voluntary control to the respective task planed, e.g. a movement. The pre-warning signal may also include vibration, a light signal, smell, taste, pain, humidity, draught, or the like.

In general, the starting sequence and/or ending sequence (thus, the ramping, i.e. the pulses of the starting sequence and/or ending sequence, with the stimulation parameters mentioned above, or the pre-pulse) may be defined either by a user (a patient, a physician, a physiotherapist, a therapist, a nurse, a family member of the patient) and/or automatically.

In particular, the starting sequence and/or ending sequence (thus, the ramping, i.e. the pulses of the starting sequence and/or ending sequence, with the stimulation parameters described above, or the pre-pulse) may be defined and/or selected automatically by the system based on functional mapping, for instance a linear mapping between motor onset and target value. Alternatively, the functional mapping could be enhanced for ramping.

The present invention also relates to the use of a system for planning and/or adjusting and/or providing a neuromodulation therapy according to any of claims 1-11 in a method for the treatment of a patient suffering from spinal cord injury, stroke, multiple sclerosis, autonomic failure, autonomic neuropathy and/or cancer of the neurological tissue.

According to the present invention a method is disclosed, the method characterized in that the method is performed with the system of any of claims 1-11.

In particular, the method may be a method for planning and/or adjusting and/or providing a neuromodulation therapy, comprising the steps of:
- providing neuromodulation at least partially by means of neurostimulation;
- controlling the neuromodulation,
wherein the neuromodulation is controlled at the beginning of a neuromodulation action including neurostimulation that the neurostimulation comprises a starting sequence and/or at the end of a neuromodulation action including neurostimulation that the neurostimulation comprises an ending sequence,
wherein the neuromodulation action is planned to be provided and/or provided to a neuronal population (NP) of a patient for neuronal population polarization,
further wherein the neuronal population polarization during the starting sequence and/or ending sequence is limited compared to the neuronal population polarization during the stimulation after the starting sequence and/or before the ending sequence.
Further details and advantages of the present invention shall now be disclosed in connection with the drawings.

It is shown in
- Fig. 1: a schematical illustration of a response to stimulation with a neurostimulation system as known in the prior art, without a starting sequence according to the present invention;
- Fig. 2: a schematical overview of an embodiment of the neuromodulation system for planning and/or adjusting and/or providing a neuromodulation therapy, according to the present invention.
- Fig. 3: an example of a starting sequence including a pre-pulse according to the present invention;
- Fig. 4: an example of a starting sequence including pulse ramping, according to the present invention;
- Fig. 5: an example of responses to a starting sequence including pulse ramping followed by responses to a standard stimulation pulse train at a fixed frequency and constant amplitude, according to the present invention.
- Fig. 6: examples of responses to pre-pulses of different amplitude, followed by responses to a standard stimulation block (pulse train) at a fixed frequency and constant amplitude, according to the present invention;
- Fig. 7: further examples of responses to pre-pulses of different amplitude, followed by responses to a standard stimulation block (pulse train) at a fixed frequency and constant amplitude, according to the present invention;
- Fig. 8a: examples of responses to two subsequent pre-pulses separated by a time interval of 25 ms and of different amplitude, according to the present invention;
- Fig. 8b: examples of responses to two subsequent pre-pulses separated by a time interval of 50 ms and of different amplitude, according to the present invention;
- Fig. 9: examples of responses to ramping over two pulses, followed by responses to a standard stimulation block (pulse train) at a fixed frequency and constant amplitude, according to the present invention;
- Fig. 10: further examples of responses to ramping over two pulses, followed by responses to a standard stimulation block (pulse train) at a fixed frequency and constant amplitude, according to the present invention;
- Fig. 11: examples of responses to ramping over one to six pulses, followed by responses to a standard stimulation block (pulse train) at a fixed frequency and constant amplitude, according to the present invention;
- Fig. 12: a further example of responses to stimulation according to the present invention that have been measured by EMG; and
- Fig. 13: an example of responses to a standard stimulation block (pulse train) at a fixed frequency and constant amplitude, measured by a goniometer, without and with ramping up.

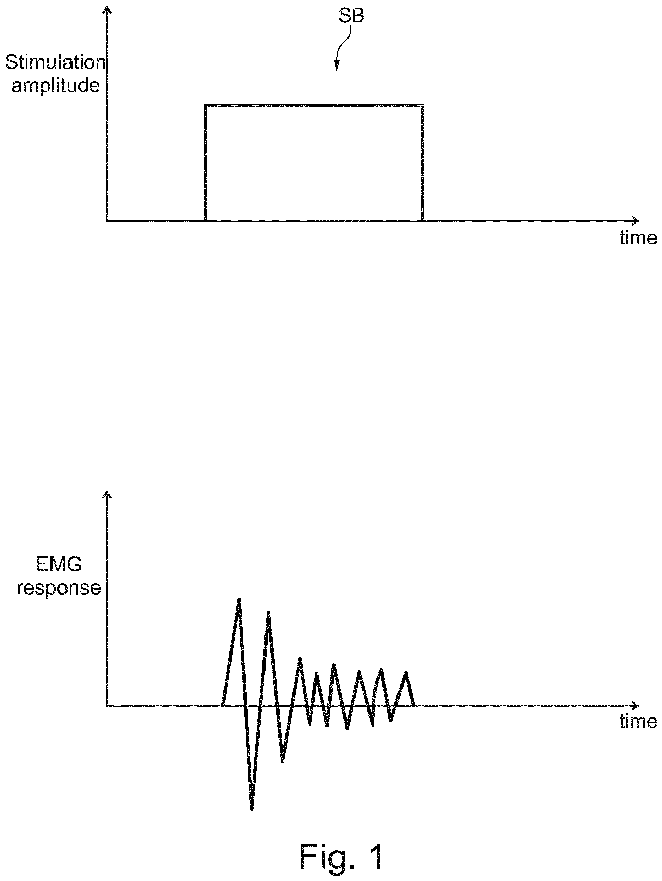

**Fig. 1** shows schematical illustration of a response to stimulation with a neurostimulation system as known in the prior art, without a starting sequence according to the present invention.

An abnormal initial response is observed, measured by e.g. EMG, after stimulation provided by a neurostimulation system.

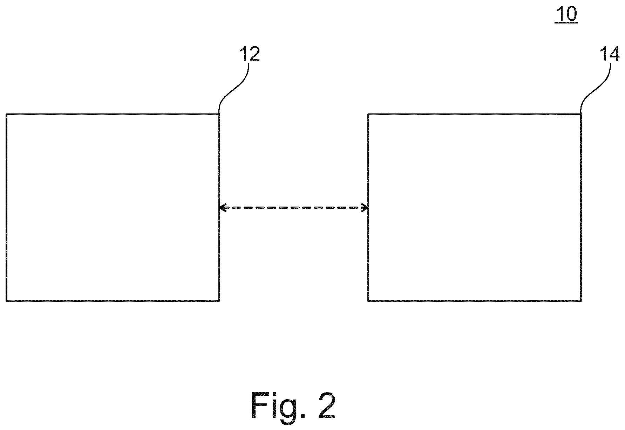

**Fig. 2** shows a schematical overview of an embodiment of the neuromodulation system 10 for planning and/or adjusting and/or providing a neuromodulation therapy, according to the present invention.

The system 10 shall reduce and/or eliminate abnormal initial responses to neuromodulation/ neurostimulation e.g. as disclosed in Fig. 1.

The system 10 comprises a neuromodulation means 12.

In general, the system 10 could comprise more than one neuromodulation means 12.

In this embodiment, the system 10 further comprises a neuromodulation controller 14.

In general, the system 10 could comprise more than one neuromodulation controller 14.

In this embodiment, the neuromodulation means 12 and the neuromodulation controller 14 are connected.

The connection between the neuromodulation means 12 and the neuromodulation controller 14 is a direct connection.

Alternatively, the connection between the neuromodulation means 12 and the neuromodulation controller 14 could be an indirect connection.

In this embodiment, the connection between the neuromodulation means 12 and the neuromodulation controller 14 is a bidirectional connection.

Alternatively, the connection between the neuromodulation means 12 and the neuromodulation controller 14 could be a unidirectional connection (from the neuromodulation means 12 to the neuromodulation controller 14 or vice versa).

In this embodiment, the connection between the neuromodulation means 12 and the neuromodulation controller 14 is a wireless connection.

Alternatively, the connection between the neuromodulation means 12 and the neuromodulation controller 14 could be a cable-bound connection.

In a system 10 comprising more than one neuromodulation means 12 and/or more than one neuromodulation controller 14, several neuromodulation means 12 and/or several neuromodulation controllers 4 could be connected.

In this embodiment, the neuromodulation means 12 provides neuromodulation by means of neurostimulation.

In an alternative embodiment, the neuromodulation means 12 could provide neuromodulation partially by means of neurostimulation.

In this embodiment, the neurostimulation means 12 provides neurostimulation to a patient.

In this embodiment, the neurostimulation comprises a starting sequence.

Alternatively, and/or additionally, the neurostimulation could comprise an ending sequence.

In this embodiment, the neurostimulation means 12 provides neurostimulation to a patient during a neuromodulation action.

In this embodiment, the neuromodulation means 14 controls the neurostimulation means 12.

Further, the neuromodulation controller 14 controls the neuromodulation means 12 at the beginning of a neuromodulation action including neurostimulation that the neurostimulation comprises a starting sequence.

Alternatively, and/or additionally, the neuromodulation controller 14 could control the neuromodulation means 12 at the end of a neuromodulation action including neurostimulation that the neurostimulation comprises an ending sequence.

In the present embodiment, the neuromodulation action is planned to be provided and/or provided to a neuronal population (NP) of a patient for neuronal population polarization.

The neuronal population polarization during the starting sequence can be limited compared to the neuronal population polarization during the stimulation after the starting sequence.

Additionally or alternatively, the neural population polarization during the starting sequence can be limited compared to the neuronal population polarization during the stimulation before the ending sequence.

Additionally or alternatively, the neuronal population polarization during the ending sequence can be limited compared to the neuronal population polarization during the stimulation after the starting sequence.

Additionally or alternatively, the neuronal population polarization during the ending sequence can be limited compared to the neuronal population polarization during the stimulation before the ending sequence.

Not shown in Fig. 2 is that in general, the neurostimulation could comprise stimulation parameters, wherein the stimulation parameters comprise power, amplitude, current, voltage, pulse width, frequency and/or duration.

Not shown in Fig. 2 is that alternatively and/or additionally, the neuromodulation could be planned to be provided and/or provided to a spatial area of a patient, wherein the spatial area during the starting sequence and/or ending sequence is limited compared to the spatial area during the stimulation after the starting sequence and/or before the ending sequence.

Further not shown in Fig. 2 is that the starting sequence and/or ending sequence could comprise a pre-warning signal and/or stop-warning signal.

The pre-warning signal and/or stop-warning signal could be an acoustic, visual, haptic, electrical, sensory and/or temperature signal.

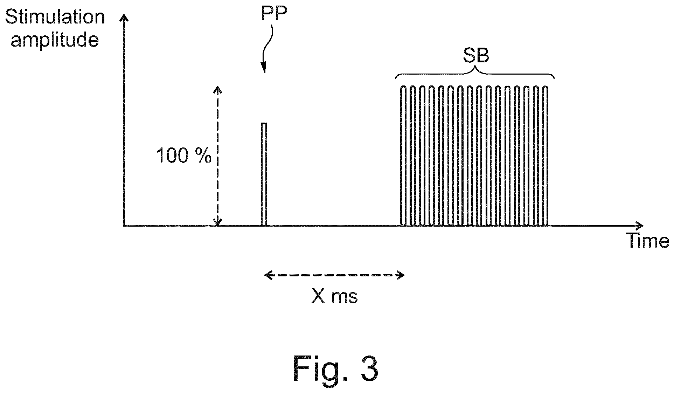

In this embodiment, the starting sequence includes a pre-pulse PP, cf. Fig. 3.

The pre-pulse PP is delivered X ms before the stimulation block SB, comprising several pulses.

In other words, one pre-pulse PP is delivered X ms before the stimulation block SB, is delivered.

In general, a pre-pulse PP could be delivered X time units before the stimulation block SB.

In general, the starting sequence can include a pre-pulse PP, wherein at least one stimulation parameter of the pre-pulse PP is of a lower level compared to the at least one stimulation parameter of a normal stimulation pulse.

In this embodiment, the starting sequence includes a pre-pulse PP, wherein the pre-pulse PP is less powerful (less amplitude) than a normal stimulation pulse (X percent of the amplitude of the normal stimulation pulses of the stimulation block SB).

In general, at least one stimulation parameter of the starting sequence and/or ending sequence could have a lower level compared to the at least one stimulation parameter of the normal stimulation.

Alternatively, the amplitude of the pre-pulse PP could be 100% percent of the amplitude of the pulses of the stimulation block SB.

In general, the parameters of the pre-pulse PP are the time interval between the pre-pulse PP and the stimulation block SB and/or its amplitude.

It could be generally possible, that the parameters of the pre-pulse comprise pulse width of the pre-pulse.

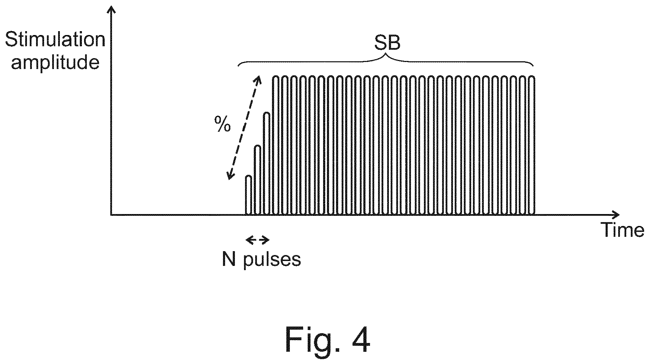

Alternatively, the starting sequence could include a pulse ramping, wherein the pulse ramping includes a ramping up from a starting pulse energy level to a higher pulse energy level being used for the neurostimulation, cf. **Fig. 4****.**

In this embodiment, the first N pulses are linearly increasing in amplitude, as a percentage of the amplitude stimulation block SB amplitude.

Not shown in Fig. 4 is that in general, the increase of at least one stimulation parameter could be a linear, non-linear, exponential, polynomic and/or stepwise increase.

In this embodiment, the starting sequence is comprised in the stimulation block SB.

However, in an alternative embodiment, the starting sequence could be not comprised in the stimulation block SB.

However, in an alternative embodiment, a non-linear increasing in the amplitude of the pulses of the ramping sequence could be generally possible.

In this embodiment, the amplitude of the first N pulses increases from 55% to 70% to 85 % of the amplitude of the stimulation block.

However, in an alternative embodiment, any other increase (linear or non-linear, in percentage of the amplitude of the stimulation block) could be possible.

In other words, the amplitude of pulses is progressively increased over N pulses.

In general, the amplitude of pulses could progressively increase.

It could be generally possible, that the starting sequences includes both, a pre-pulse PP as disclosed in Fig. 3 and a ramping as disclosed in Fig. 4, wherein the pre-pulse PP is either provided before the ramping or after the ramping.

Not shown in Fig. 4 is that additionally and/or alternatively, there could be an ending sequence.

Further not shown in Fig. 4 is that at least one stimulation parameter could be decreased during the ending sequence.

Further not shown in Fig. 4 is that, additionally and/or alternatively to the ramping up, a ramping down from a level of at least one stimulation parameter used for the neurostimulation to a lower level of the at least one stimulation parameter, below the threshold used for neurostimulation, could be possible.

In general, the starting sequence and/or the ending sequence can include a pulse ramping.

In general, at least one stimulation parameter could be increased during the starting sequence and/or decreased during the ending sequence.

Not shown in Fig. 4 is that there could be a linear, non-linear, exponential, polynomic and/or stepwise decrease of at least one stimulation parameter in an ending sequence.

In this embodiment, the system 10 for planning and/or adjusting and/or providing a neuromodulation therapy is used in a method for the treatment of a patient.

In this embodiment, the system 10 for planning and/or adjusting and/or providing a neuromodulation therapy is used in a method for the treatment of a patient suffering from spinal cord injury.

Alternatively, and/or additionally, the system 10 could be used in a method for the treatment of a patient suffering from stroke, multiple sclerosis, autonomic failure, autonomic neuropathy and/or cancer of the neurological tissue.

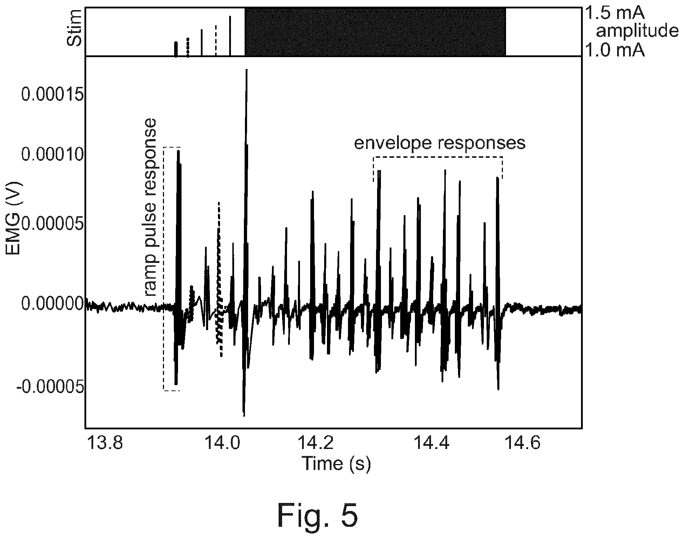

Fig. 5 shows an example of responses to a starting sequence including pulse ramping followed by responses to a standard stimulation pulse train at a fixed frequency and constant amplitude, measured by EMG.

In this embodiment, a patient is stimulated with the system 10 disclosed in Fig. 2.

In this embodiment, the starting sequence includes a pulse ramping, wherein the pulse ramping includes a ramping up from a starting pulse energy level to a higher pulse energy level being used for the neurostimulation (amplitude of 1.5 mA).

In general, any other amplitude(s) usually applied for neuromodulation could be possible.

The amplitude, which could be understood as pulse energy level, is continuously increasing during ramping from the starting pulse to the amplitude used during stimulation.

The one skilled could understand that the initial stimulation causes a shock-like situation for the muscle targeted, as the first response to the fist stimulation pulse is high compared to the following pulses.

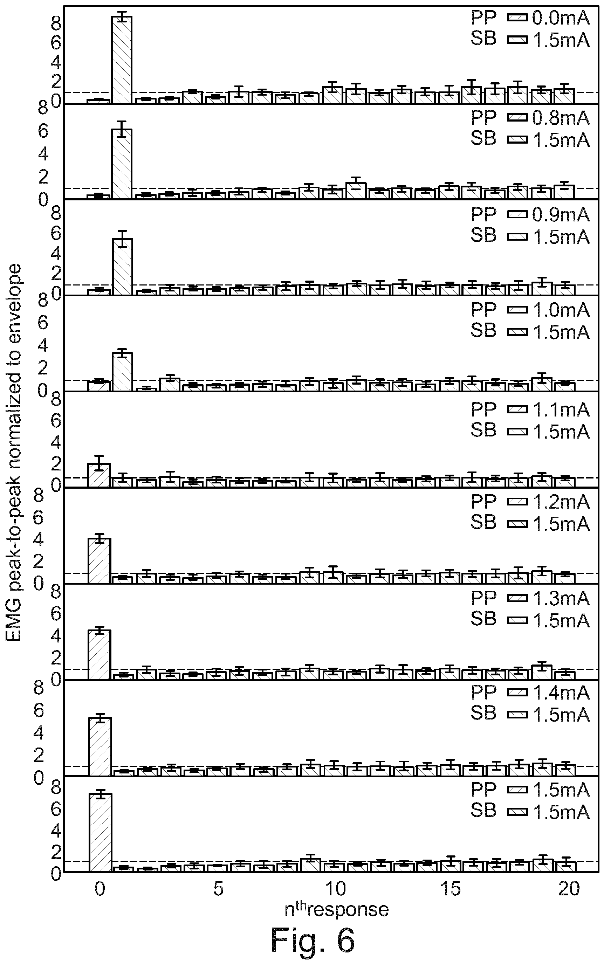

**Fig. 6** shows examples of responses to pre-pulses of different amplitude, followed by responses to a standard stimulation block SB (pulse train) at a fixed frequency and constant amplitude, measured by EMG.

In particular, responses to a single pre-pulse PP at an amplitude of 0.0 mA, 0.8 mA, 0.9 mA, 1.0 mA, 1.1 mA, 1.2 mA, 1.3 mA, 1.4 mA, and 1.5 mA, followed by responses to a standard stimulation block SB (pulse train) at a fixed frequency and constant amplitude of 1.5 mA, measured by EMG, are shown.

In this embodiment, the right tibialis anterior TA was targeted by neurostimulation with the system 10 according to Fig. 2.

In this embodiment, the responses of stimulation were measured by EMG at the right tibialis anterior TA.

In this embodiment, the pre-pulse PP was set 25 ms before the stimulation block SB (pulse train).

However, any other time interval between the pre-pulse PP and the stimulation block sB could be generally possible.

In this embodiment, a stimulation at 1.1 mA (corresponding to 73% of the target amplitude) shows the optimal result (maximal suppression of overshoot).

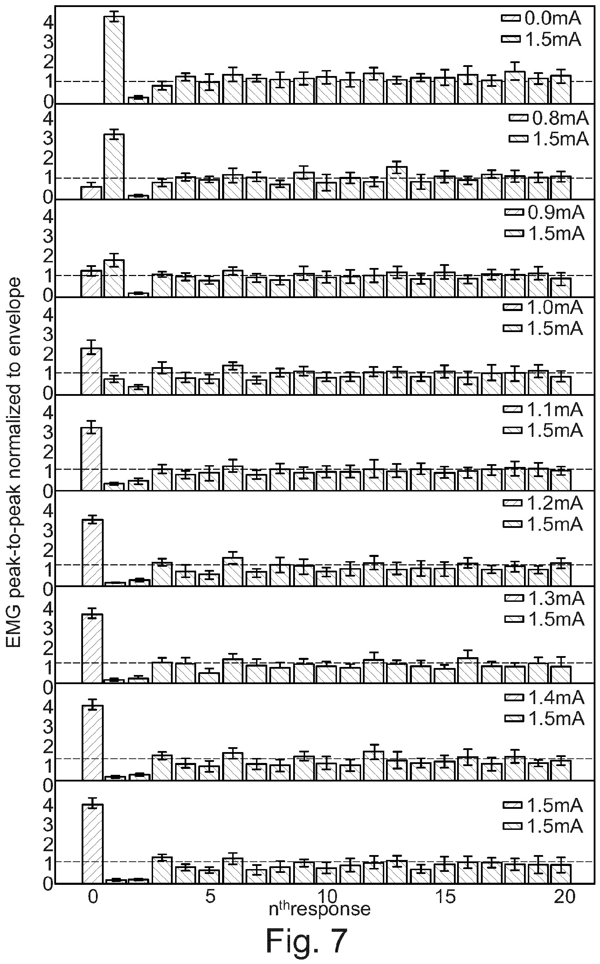

**Fig. 7** shows further examples of responses to pre-pulses PP of different amplitude, followed by responses to a standard stimulation block SB (pulse train) at a fixed frequency and constant amplitude, measured by EMG.

In particular, responses to a single pre-pulse PP at an amplitude of 0.0 mA, 0.8 mA, 0.9 mA, 1.0 mA, 1.1 mA, 1.2 mA, 1.3 mA, 1.4 mA, and 1.5 mA, followed by responses to a standard stimulation block SB (pulse train) at a fixed frequency and constant amplitude of 1.5 mA, measured by EMG, are shown.

In this embodiment, the right tibialis anterior TA was targeted by neurostimulation with the system according to Fig. 2.

In this embodiment, the responses of stimulation were measured by EMG at the right medial gastrocnemius MG.

In this embodiment, the pre-pulse was set 25 ms before the stimulation block SB (pulse train).

However, any other time interval (seconds or milliseconds) between the pre-pulse PP and the stimulation block SB could be generally possible.

In this embodiment, a stimulation at 0.9 mA (corresponding to 60% of the target amplitude) shows the optimal result (maximal suppression of overshoot).

In this embodiment, this results in an almost complete suppression of the third pulse (2 pulses after pre-pulse).

In this embodiment, the optimal amplitude for the target muscle (1.1 mA) already leads to a relatively large overshoot.

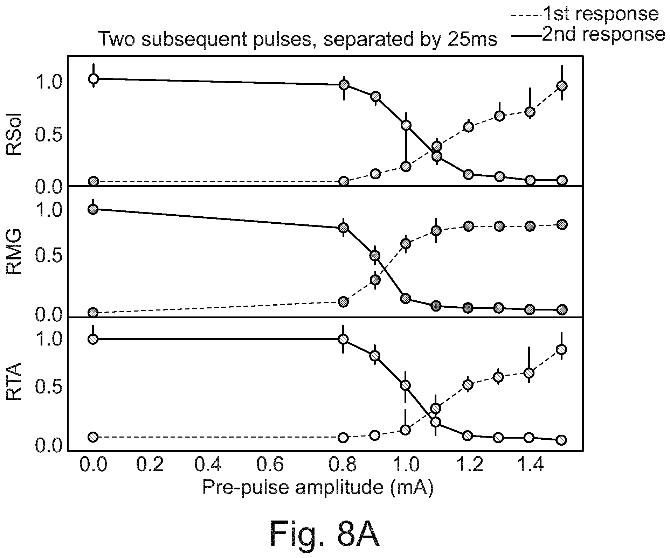

**Fig. 8a** shows examples of responses to two subsequent pre-pulses PP separated by a time interval of 25 ms and of different amplitude, measured by EMG.

In particular, responses to a two subsequent pre-pulse PP with an amplitude of 0.0 mA, 0.8 mA, 0.9 mA, 1.0 mA, 1.1 mA, 1.2 mA, 1.3 mA, 1.4 mA, and 1.5 mA, measured by EMG, are shown.

In this embodiment, the right tibialis anterior TA was targeted by neurostimulation with the system 10 according to Fig. 2.

In this embodiment, the responses of stimulation were measured by EMG at the right tibialis anterior TA, the right soleus and the right medial gastrocnemius MG.

In this embodiment, the time interval between the two pre-pulses PP was set 25 ms.

The response to the first pre-pulse PP is indicated by the dashed line.

The response to the second pre-pulse PP is indicated by the solid line.

However, any other time interval between the two pre-pulses PP could be generally possible.

The optimum amplitude for the pre-pulse PP is at the crossing of the two lines, where response due to first pre-pulse PP is equal to the response due to the second pre-pulse PP.

In this embodiment, different muscles respond best to different amplitudes and the optimal amplitude changes with the timing of the pre-pulse PP.

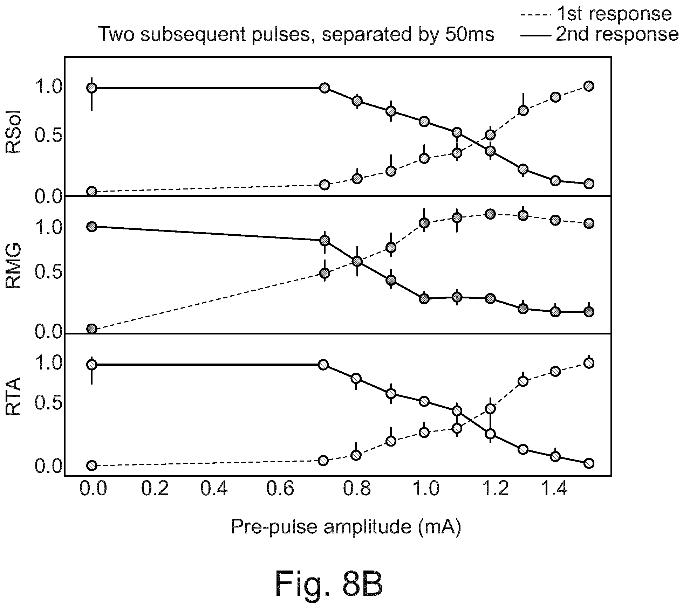

**Fig. 8b** shows examples of responses to two subsequent pre-pulses PP separated by a time interval of 50 ms and of different amplitude, measured by EMG.

In particular, responses to a two subsequent pre-pulses PP with an amplitude of 0.0 mA, 0.7 mA, 0.8 mA, 0.9 mA, 1.0 mA, 1.1 mA, 1.2 mA, 1.3 mA, 1.4 mA, and 1.5 mA, measured by EMG, are shown.

In this embodiment, the right tibialis anterior TA was targeted by neurostimulation with the system 10 according to Fig. 2.

In this embodiment, the responses to stimulation were measured by EMG at the right tibialis anterior TA, the right soleus and the right medial gastrocnemius MG.

In this embodiment, the time interval between the two pre-pulses PP was set 50 ms.

However, any other time interval (seconds or milliseconds) between the two pre-pulses could be generally possible.

The response to the first pre-pulse PP is indicated by the dashed line.

The response to the second pre-pulse PP is indicated by the solid line.

The optimum amplitude for the pre-pulse PP is at the crossing of the two lines, where response due to first pre-pulse PP is equal to the response due to the second pre-pulse PP.

In this embodiment, different muscles respond best to different amplitudes and the optimal amplitude changes with the timing of the pre-pulse PP.

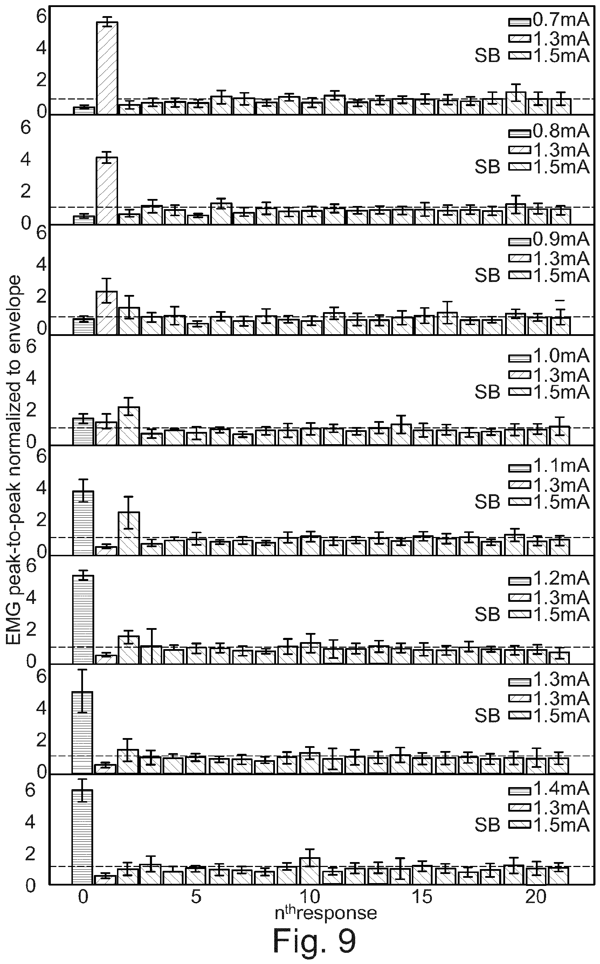

**Fig. 9** shows examples of responses to ramping over two pulses, followed by responses to a standard stimulation block SB (pulse train) at a fixed frequency and constant amplitude, measured by EMG.

In this embodiment, responses to ramping over two pulses (first pulse of ramping with an amplitude of 0.7 mA, 0.8 mA, 0.9 mA, 1.0 mA, 1.1 mA, 1.2 mA, 1.3 mA, or 1.4 mA; second pulse of ramping with an amplitude of 1.3 mA), followed by responses to a standard stimulation block (SB pulse train) at a fixed frequency and constant amplitude of 1.5 mA, measured by EMG, are shown.

In this embodiment, the right tibialis anterior TA was targeted by neurostimulation with the system 10 according to Fig. 2.

In this embodiment, the responses of stimulation were measured by EMG at the right tibialis anterior RTA.

The first pulse must be sufficiently strong to have any effect (see upper plots).

In case the amplitude of the first plot is too high it induces the same muscle response as an onset without ramps (see lower plots).

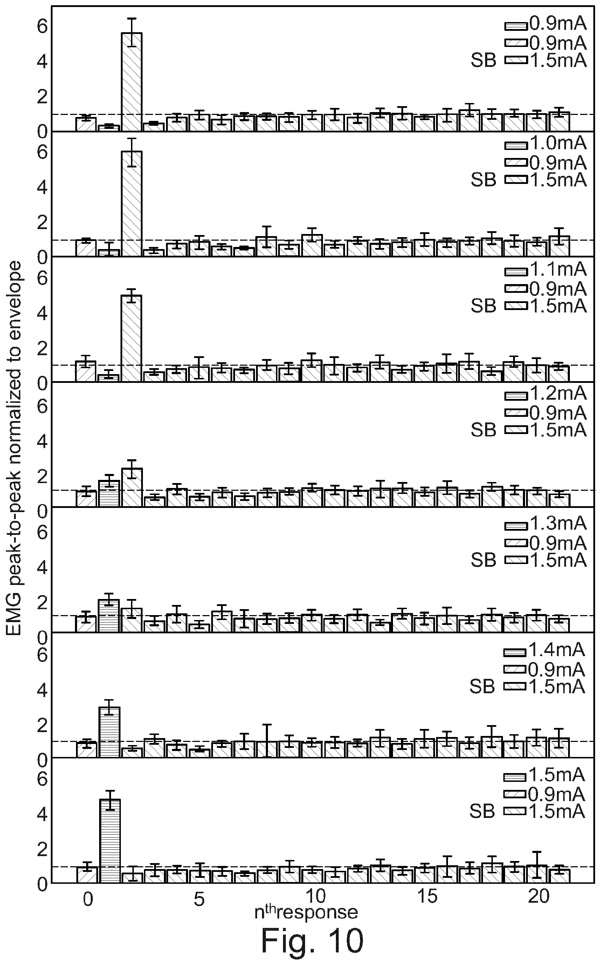

**Fig. 10** shows further examples of responses to ramping over two pulses, followed by responses to a standard stimulation block SB (pulse train) at a fixed frequency and constant amplitude, measured by EMG.

In this embodiment, responses to ramping over two pulses (first pulse of ramping with an amplitude of 0.9 mA, second pulse of ramping with an amplitude of 0.9 mA, 1.0 mA, 1.1 mA, 1.2 mA, 1.3 mA, 1.4 mA, or 1.5 mA), followed by responses to a standard stimulation block SB (pulse train) at a fixed frequency and constant amplitude of 1.5 mA, measured by EMG, are shown.

In this embodiment, the right tibialis anterior TA was targeted by neurostimulation with the system 10 according to Fig. 2.

In this embodiment, the responses of stimulation were measured by EMG at the right tibialis anterior RTA.

The first pulse must be sufficiently strong to have any effect (see upper plots).

If ramping pulses are chosen too close to each other (in amplitude), the first ramp pulse could lead to a suppression of the response to the 2^{nd} ramp pulse and therefore cancel out the ramping effect (e.g. 3^{rd} plot from the top).

If the ramping pulses too far apart (in amplitude), the effect of the second pulse could be too strong (bottom plot).

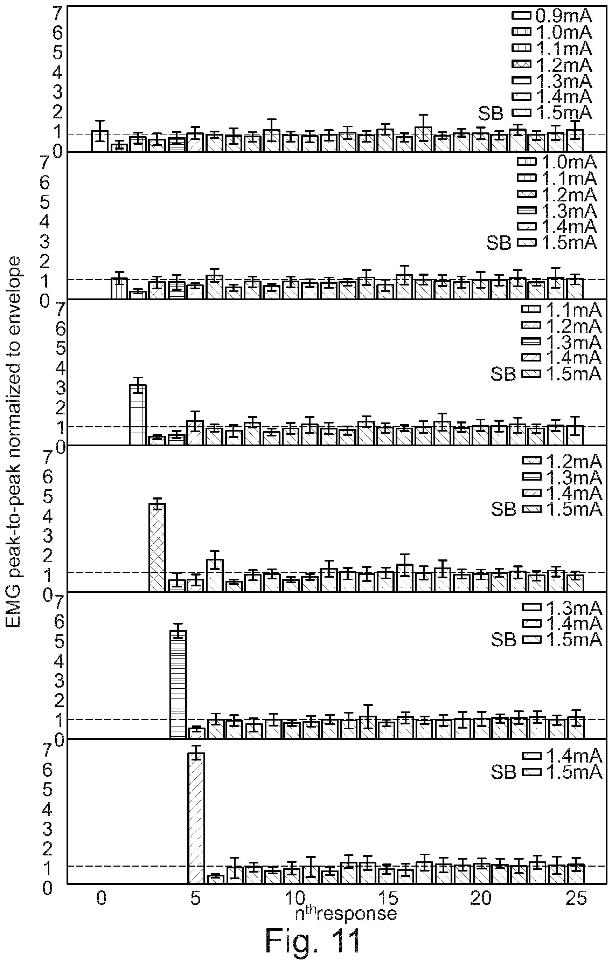

**Fig. 11** shows examples of responses to ramping over one to six pulses, followed by responses to a standard stimulation block SB (pulse train) at a fixed frequency and constant amplitude, measured by EMG.

In this embodiment, responses to ramping over one two six pulses (first pulse of ramping with an amplitude of 0.9 mA, 1.0 mA, 1.1 mA, 1.2 mA, 1.3 mA, or 1.4 mA; second pulse of ramping with an amplitude of 1.0 mA, 1.1 mA, 1.2 mA, 1.3 mA, or 1.4 mA; third pulse of ramping with an amplitude 1.1 mA, 1.2 mA, 1.3 mA, or 1.4 mA, fourth pulse of ramping with an amplitude of 1.2 mA, 1.3 mA, or 1.4 mA; fifth pulse of ramping with an amplitude of 1.3 mA, or 1.4 mA; six pulse of ramping with an amplitude of 1.4 mA), followed by responses to a standard stimulation block SB (pulse train) at a fixed frequency and constant amplitude of 1.5 mA, measured by EMG, are shown.

In this embodiment, the right tibialis anterior TA was targeted by neurostimulation with the system 10 according to Fig. 2.

In this embodiment, the responses of stimulation were measured by EMG at the right tibialis anterior RTA.

In this embodiment, it is started with six pulses of ramping (top) and then consequently one pulse is removed from the ramping sequence.

Also, for multiple pulses, the correct choice of amplitude for the first pulse is essential to benefit from the ramps.

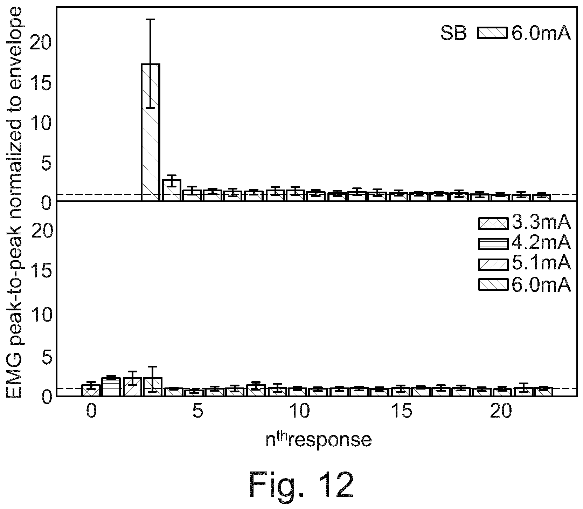

**Fig. 12** shows a further example of responses to stimulation according to the present invention that have been measured by EMG.

In this embodiment, the right tibialis anterior TA was targeted by neurostimulation with the system 10 according to Fig. 2.

In this embodiment, the responses of stimulation were measured by EMG at the right tibialis anterior TA.

On top of Fig. 12, responses to a standard stimulation block SB (pulse train) at a fixed frequency and constant amplitude, measured by EMG, are shown, without ramping up.

On the bottom of Fig. 12, responses to up-ramping with three pulses, followed by responses to a standard stimulation block SB (pulse train) at a fixed frequency and constant amplitude, measured by EMG, are shown.

In particular, up-ramping with pulses of 55%, 70% and 85% of the amplitude of the pulses of the stimulation block are shown.

Alternatively, up-ramping with three pulses of 40-60%, 60-80% and 80-100% of the amplitude of the pulses of the stimulation block SB could be generally possible.

In this embodiment, the amplitude of the pulses of the stimulation block SB is 6.0 mA.

However, in an alternative embodiment, the amplitude of the pulses of the stimulation block could be 0.2mA to 200 mA.

In this embodiment, the up-ramping sequence causes significant reduction of the initial response peak.

In general, the up-ramping sequence may comprise two or more pulses.

Not shown in Fig. 12 is that the amplitude of the pulses of up-ramping (independent of the number of pulses of the up-ramping) may be of any amplitude lower or equal to the amplitude of the pulses of the stimulation block SB.

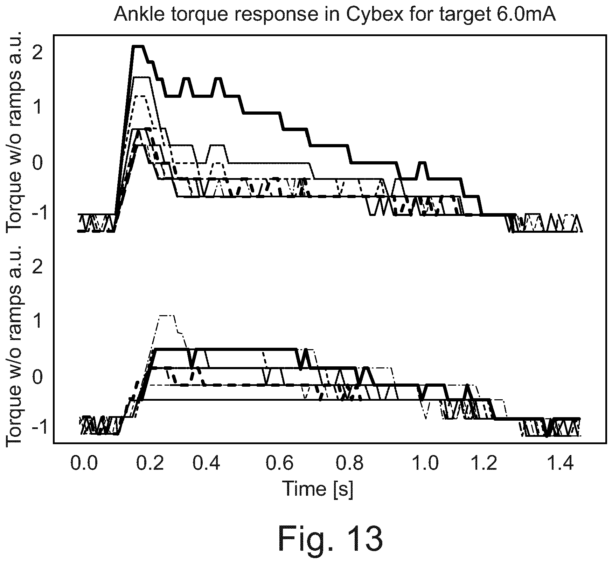

**Fig. 13** shows responses to a standard stimulation block SB (pulse train) at a fixed frequency and constant amplitude, measured by a goniometer, without and with ramping up.

In Fig. 13, responses to stimulation according to the present invention are shown that have been measured by a goniometer.

In this embodiment, the right tibialis anterior TA was targeted by neurostimulation with the system 10 according to Fig. 2.

On top of Fig. 13, responses to a standard stimulation block SB (pulse train) at a fixed frequency and constant amplitude are shown, without ramping up (no starting sequence).

On the bottom of Fig. 13, responses to up-ramping with three pulses of, followed by responses to a standard stimulation block SB (pulse train) at a fixed frequency and constant amplitude, measured by EMG, are shown.

In particular, up-ramping with pulses of 55%, 70% and 85% of the amplitude of the pulses of the respective stimulation block SB are shown.

In general, the amplitude of the pulses of up-ramping may be of any amplitude lower or equal to the amplitude of the pulses of the stimulation block SB.

In this embodiment, the up-ramping sequence causes significant reduction/elimination of the initial response peak (as illustrated in the bottom graph).

### References

- 10: system
- 12: neuromodulation means
- 14: neuromodulation controller

- PP: pre-pulse
- SB: stimulation block

## Claims

1. A neuromodulation system (10) for planning and/or adjusting and/or providing a neuromodulation therapy, comprising:
- at least one neuromodulation means (12) configured to provide neuromodulation at least partially by means of neurostimulation;
- at least one neuromodulation controller (14) configured to control the neuromodulation means (12),
wherein the neuromodulation controller (14) is further configured to control the neuromodulation means (12) at the beginning of a neuromodulation action including neurostimulation that the neurostimulation comprises a starting sequence and/or at the end of a neuromodulation action including neurostimulation that the neurostimulation comprises an ending sequence,
wherein the neuromodulation action is planned to be provided and/or provided to a neuronal population (NP) of a patient for neuronal population polarization, and
wherein the neuronal population polarization during the starting sequence and/or ending sequence is limited compared to the neuronal population polarization during the stimulation after the starting sequence and/or before the ending sequence.

2. The neuromodulation system (10) according to claim 1, **characterized in that** the neurostimulation comprises stimulation parameters, wherein the stimulation parameters comprise power, amplitude, current, voltage, pulse width, frequency and/or duration.

3. The neuromodulation system (10) according to claim 2, **characterized in that** at least one stimulation parameter of the starting sequence and/or ending sequence has a lower level compared to the at least one stimulation parameter of the normal stimulation.

4. The neuromodulation system (10) according to claim 1, claim 2 or claim 3, **characterized in that** at least one stimulation parameter is increased during the starting sequence and/or decreased during the ending sequence.

5. The neuromodulation system (10) according to claim 4, **characterized in that** the increase the at least one stimulation parameter is a linear, non-linear, exponential, polynomic and/or stepwise increase and that the decrease of the at least one stimulation parameter is a linear, non-linear, exponential, polynomic and/or stepwise decrease.

6. The neuromodulation system (10) according to one of the preceding claims, **characterized in that** the starting sequence includes a pre-pulse (PP), wherein at least one stimulation parameter of the pre-pulse (PP) is of a lower level compared to the at least one stimulation parameter of a normal stimulation pulse.

7. The neuromodulation system (10) according to one of the preceding claims, **characterized in that** the starting sequence and/or the ending sequence includes a pulse ramping.

8. The neuromodulation system (10) according to claim 7, **characterized in that** the pulse ramping includes a ramping up from a starting level of at least one stimulation parameter to a higher level of the at least one stimulation parameter used for the neurostimulation and/or a ramping down from a level of at least one stimulation parameter used for the neurostimulation to a lower level of the at least one stimulation parameter, below the threshold used for neurostimulation.

9. The system (10) according to any of the preceding claims, **characterized in that** the level of at least one stimulation parameter of the starting sequence and/or ending sequence is determined manually and/or automatically based on response data.

10. The neuromodulation system (10) according to any of the preceding claims, **characterized in that** the starting sequence and/or ending sequence comprises a pre-warning signal and/or stop-warning signal.

11. The neuromodulation system (10) according to claim 10, **characterized in that** the pre-warning signal and/or stop-warning signal is an acoustic, visual, haptic, electrical, sensory and/or temperature signal.

12. The use of a system (10) for planning and/or adjusting and/or providing a neuromodulation therapy according to any of claims 1-11 in a method for the treatment of a patient suffering from spinal cord injury, stroke, multiple sclerosis, autonomic failure, autonomic neuropathy and/or cancer of the neurological tissue.
